(19) Europäisches Patentamt
European Patent Office
Office européen des brevets

(11) Veröffentlichungsnummer : **0 325 126 B1**

(12) # EUROPÄISCHE PATENTSCHRIFT

(45) Veröffentlichungstag der Patentschrift : **06.03.91 Patentblatt 91/10**

(51) Int. Cl.$^5$ : **C07F 9/06**

(21) Anmeldenummer : **89100237.0**

(22) Anmeldetag : **07.01.89**

(54) **Fluor enthaltende 1-Arylalkoxytris(dialkylamino)-phosphonium-salze, Verfahren zu deren Herstellung und ihre Verwendung.**

(30) Priorität : **18.01.88 DE 3801248**

(43) Veröffentlichungstag der Anmeldung : **26.07.89 Patentblatt 89/30**

(45) Bekanntmachung des Hinweises auf die Patenterteilung : **06.03.91 Patentblatt 91/10**

(84) Benannte Vertragsstaaten : **CH DE FR GB IT LI NL**

(56) Entgegenhaltungen :
**JP-A- 5 610 121**
**US-A- 3 408 411**

(73) Patentinhaber : **HOECHST AKTIENGESELLSCHAFT**
**Postfach 80 03 20**
**W-6230 Frankfurt am Main 80 (DE)**

(72) Erfinder : **Kruse, Alfred, Dr.**
**Hornauer Strasse 199**
**W-6233 Kelkheim (Taunus) (DE)**
Erfinder : **Siegemund, Günter, Dr.**
**Frankfurter Strasse 21**
**W-6238 Hofheim am Taunus (DE)**
Erfinder : **Schumann, Axel**
**Willi-Haas-Strasse 34**
**W-5305 Alfter 3 (DE)**
Erfinder : **Ruppert, Ingo Walter, Dr.**
**(verstorben) (DE)**

**EP 0 325 126 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft 1-Arylalkoxy-tris(dialkylamino)phosphoniumsalze der Formel I

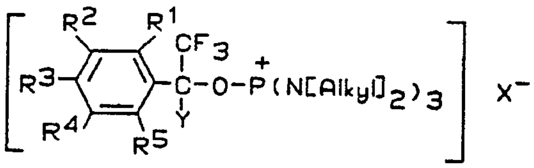

(I)

sowie ein Verfahren zu deren Herstellung und deren weitere Umsetzung zu aromatischen Verbindungen mit einer teilfluorierten Seitenkette.

Als Ausgangsmaterialien werden aromatische Aldehyde oder Ketone eingesetzt, die durch Reaktion mit Trifluormethylhalogeniden und Phosphorigsäuretriamiden in Phosphoniumsalze der vorstehenden Formel I übergeführt werden.

Es ist bekannt, Alkoxy-tris(dialkylamino)phosphoniumsalze durch Umsetzung der entsprechenden Alkohole mit einem reaktiven Halogen-tris(dialkylamino)phosphoniumsalz herzustellen (Synthesis 1979, 951-2).

Die Herstellung von trifluormethylsubstituierten Carbinolen, den der Phosphoniumsalzen der Formel I zugrundeliegenden Alkoholen, durch Übertragung des Trifluormethylrestes auf Carbonylverbindungen ist von großem Interesse und in vielen Publikationen untersucht worden. Verwendet werden dabei Organometallverbindungen unedler Metalle, die gewöhnlich aus den entsprechenden Trifluormethylhalogeniden und einem Metall, wie Magnesium, Zink, Mangan usw. hergestellt werden. Hier sind die in der Regel aufwendige Herstellung und die Labilität der zunächst herzustellenden Organometallverbindungen von Nachteil, was sich auch durch schlechte Reproduzierbarkeit publizierter Ergebnisse bemerkbar macht (Tetrahedron Lett. 26, 5243 bis 5246 ; speziell S. 5245 Fußnote 4).

Gegenstand der Erfindung sind nun Verbindungen der Formel I (s. oben), worin die Reste $R^1$ bis $R^5$ gleich oder verschieden sind und Wasserstoff, Alkyl mit 1 bis 6 Kohlenstoffatomen, das perfluoriert sein kann, Alkoxy- oder Alkylthio mit jeweils 1 bis 6, insbesondere 1 bis 3 Kohlenstoffatomen, sowie Halogen (Fluor, Chlor, Brom, Jod) bedeuten, wobei jedoch nicht mehr als drei der Reste $R^1$ bis $R^5$ eine andere Bedeutung als Wasserstoff haben, Y Wasserstoff oder einen Perfluoralkylrest $C_nF_{2n+1}$ mit 1 bis 6 Kohlenstoffatomen bedeutet, X Brom oder Jod ist und "Alkyl" für einen Alkylrest mit 1 bis 3 Kohlenstoffatomen steht.

An den aromatischen Ring sind vorzugsweise höchstens zwei Substituenten $R^1$ bis $R^5$ mit anderer Bedeutung als Wasserstoff gebunden. Die Alkyl-, Alkoxy- und Alkylthiosubstituenten können geradkettig oder verzweigt sein und enthalten zweckmäßig zusammen höchstens 6, insbesondere höchstens 4 C-Atome.

Gegenstand der Erfindung ist auch ein einfaches, einstufiges Verfahren zur Herstellung der vorgenannten Verbindungen. Dies gelingt durch Übertragung einer Trifluormethylgruppe auf aromatische Carbonylverbindungen unter Vermeidung der Herstellung und Verwendung der zuvor angeführten Organometallverbindungen und besteht darin, daß man Carbonylverbindungen der allgemeinen Formel II

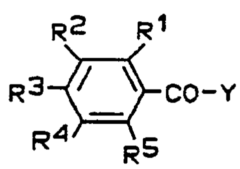

(II)

mit Trifluormethylhalogeniden der Formel $CF_3X$ (III), wobei X Brom oder Jod ist, und Phosphorigsäuretrisdialkylamiden (in anderen Worten Tris(dialkylamino)phosphanen) der allgemeinen Formel $P(N[Alkyl]_2)_3$ (IV) zu Phosphoniumsalzen der Formel I (s. oben) umsetzt, wobei in den Formeln I bis III die Reste $R^1$ bis $R^5$, Y und "Alkyl" die oben angegebene Bedeutung haben. Diese Phosphoniumsalze sind für Synthesen sehr nützliche Zwischenprodukte und können – wie später gezeigt wird – in Aromaten mit teilfluorierten Seitenketten übergeführt werden, die sonst oft nur schwer auf anderen Wegen zugänglich sind.

Das erfindungsgemäße Verfahren hat nicht nur den Vorteil der Einfachheit, sondern auch den, daß die Ausgangsmaterialien gut zugänglich sind und daß durchweg gute Ausbeuten an Phosphoniumsalzen erhalten werden. Für die Übertragung des Trifluormethylrestes auf die Carbonylverbindung kann hier mit Vorteil das gegenüber Trifluormethyljodid weniger giftige und billigere Trifluormethylbromid eingesetzt werden.

Als aromatische Carbonylverbindungen (II) können die Aldehyde (Y = Wasserstoff) oder Arylperfluoralkylketone (Y = Perfluoralkylrest $C_nF_{2n+1}$ mit n = 1 bis 6) eingesetzt werden. Die aromatischen Carbonylverbindungen können unsubstituiert sein oder einen oder mehrere gleiche oder unterschiedliche Substituenten $R^1$ bis $R^5$ mit anderer Bedeutung als Wasserstoff tragen. Als Phosphorigsäuretrisdialkylamide (IV) kommen z.B. in Frage das Trisdimethylaminophosphan, Trisdiäthylaminophosphan und Trisdipropyl- bzw. -isopropylaminophosphan ; bevorzugt wird Trisdiäthylaminophosphan $P(N[CH_2CH_3]_2)_3$ verwendet. Dieses läßt sich sehr einfach in hohen Ausbeuten durch Reaktion von Phosphortrichlorid mit Diäthylamin in einem gegenüber den Reaktionsteilnehmern inerten Lösungsmittel, z.B. einem aliphatischen, cycloaliphatischen oder aromatischen Kohlenwasserstoff oder einem Kohlenwasserstoffgemisch, erzeugen. Die Dialkylaminogruppen können gleiche oder verschiedene Alkylgruppen enthalten.

Bei der Umsetzung der aromatischen Aldehyde oder Ketone (II) mit einem Trifluormethylhalogenid (III) und Phosphorigsäuretrisdialkylamid (IV) wird zunächst ein Addukt der Formel (VI) gebildet

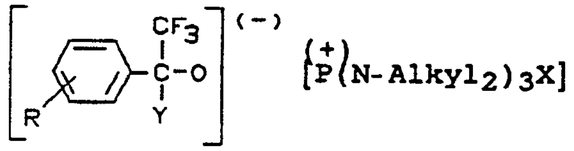 (VI).

Die Existenz dieser Verbindung und die Zuordnung der Struktur VI wird aus dem beobachteten Reaktionsverhalten plausibel. Diese Verbindung unterscheidet sich von den Verbindungen I durch ihre Reaktivität mit Carbonsäurehalogeniden unter Bildung von Estern und auch dadurch, daß sie durch Zugabe einer Protonensäure in den freien Alkohol übergeführt wird. Die erfindungsgemäßen Verbindungen I sind diesen Reaktionen nicht zugänglich. Die zunächst gebildeten Addukte VI unterliegen in der Reaktionsmischung oberhalb einer Umwandlungstemperatur, die je nach Art des zugrundeliegenden Alkohols zwischen −60°C und +20°C liegt, einer exothermen Umlagerung in die erfindungsgemäßen Arylalkoxy-tris(dialkylamino)phosphonium-Salze (I).

Die Umsetzung der Carbonylverbindungen mit dem Trifluormethylhalogenid und Phosphorigsäuretrisdialkylamid wird im allgemeinen bei Temperaturen von etwa −100°C bis +50°C, insbesondere von −80 bis +20°C durchgeführt. Bei sehr wenig reaktiven Carbonylverbindungen ist es zweckmäßig, bei Temperaturen oberhalb −40°C und z.B. bis +50°C zu arbeiten, um eine zügige Umsetzung zu erreichen. Die Reaktionsdauer ist bekanntlich von den übrigen Bedingungen, insbesondere von der Reaktionstemperatur, abhängig. Die Reaktion ist im allgemeinen im Zeitraum von wenigen Minuten bis zu mehreren Stunden beendet.

Die Umsetzung wird im allgemeinen ohne Anwendung von Überdruck ausgeführt. Es kann jedoch zweckmäßig sein, bei erhöhtem Druck zu arbeiten, vor allem wenn oberhalb der Siedetemperatur (bei Atmosphärendruck) des Trifluormethylhalogenids gearbeitet wird. Praktisch wird man dann also bei mindestens dem Eigendruck arbeiten.

Zweckmäßig wird das vorliegende Verfahren unter wasserfreien Bedingungen in Gegenwart eines gegenüber den Reaktionsteilnehmern inerten Lösungs- oder Verdünnungsmittels durchgeführt. Als solche kommen vor allem aprotische Flüssigkeiten zum Einsatz. Verwendet werden z.B. halogenierte Kohlenwasserstoffe wie Methylenchlorid, Tetrachloräthan, Nitrile, z.B. Acetonitril oder dessen Homologe wie Buttersäurenitril, oder Benzonitril, Ester wie Diäthylcarbonat oder Äthylencarbonat und Äther wie Tetrahydrofuran oder Dimethoxyäthan. Das Lösungsmittel sollte möglichst wasserfrei sein.

Es ist von Vorteil, während der gesamten Dauer der Reaktion, z.B. durch Rühren, für eine gute Durchmischung des Ansatzes zu sorgen sowie durch Wahl eines geeigneten Lösungsmittels das Reaktionsprodukt in Lösung zu halten.

Die Art und die Reihenfolge der Vereinigung der drei Komponenten ist nicht kritisch. Das erfindungsgemäße Verfahren kann beispielsweise so durchgeführt werden, daß man das Lösungsmittel, die Carbonylverbindung und eine weitere Komponente vorlegt und die dritte zudosiert. Es können auch alle drei Komponenten gleichzeitig vereinigt werden. Die anderen Reaktionsteilnehmer werden gewöhnlich in einer zur Carbonylverbindung II mindestens äquivalenten Menge, oft aber in einem Überschuß von z.B. bis zu 25% eingesetzt.

Die Aufarbeitung kann z.B. erfolgen, indem man das Reaktionsgemisch unter vermindertem Druck vom Lösungsmittel befreit und den resultierenden Rückstand umkristallisiert. Für die Isolierung der Phosphoniumsalze kann es zweckmäßig sein, zunächst Nebenprodukte und einen Teil des Lösungsmittels durch Extraktion der Reaktionsmischung mit einem unpolaren Lösungsmittel, z.B. einem Kohlenwasserstoff wie Hexan, zu entfernen. Bei dieser Operation Fällt die untere Phase, die im wesentlichen das Phosphoniumsalz I enthält, oft schon als Feststoff an.

Die erfindungsgemäßen Phosphoniumsalze sind recht stabile, hydrolysebeständige Feststoffe, gut löslich in Wasser und polaren Lösungsmitteln. Sie sind weiterhin präparativ sehr nützliche Verbindungen, die sich leicht in einem Schritt zu anderen interessanten aromatischen Verbindungen mit teilfluorierten Seitenketten umwandeln lassen. So erfolgt beim Erhitzen der Phosphoniumsalze I eine Spaltung der Kohlenstoff-Sauerstoff-Bindung am ehemaligen Carbonylkohlenstoffatom, und es wird unter Substitution durch das Halogenid-Ion ein Molekül Phosphorsäuretriamid $P(O)(N[Alkyl]_2)_3$ abgespalten. Dabei werden an sich bekannte aromatische Verbindungen der allgemeinen Formel V

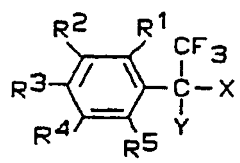

(V),

gebildet, die in der fluorierten Seitenkette in der α-Position Brom oder Jod enthalten und in denen $R^1$ bis $R^5$ und Y die obengenannten Bedeutungen haben. Diese Spaltung verläuft in vielen Fällen nahezu quantitativ. Dazu wird das Phosphoniumsalz in Substanz oder einem inerten Lösungsmittel, z.B. solchen mit einem Siedepunkt von mindestens der Schmelztemperatur des Phosphoniumsalzes, wie Methylisobutylketon, Tetrahydronaphthalin, in der Regel auf Temperaturen oberhalb des Schmelzpunktes erhitzt. Bei Verwendung eines Lösungsmittels tritt die Umwandlung schon bei Temperaturen unterhalb des Schmelzpunktes ein. Bei einzelnen Phosphoniumsalzen, z.B. dem Produkt aus Beispiel 5, können auch recht niedrig siedende Lösungsmittel verwendet werden, wie Aceton. Die Reaktionsbedingungen sind nicht kritisch ; die beiden Reaktionsprodukte lassen sich leicht durch Destillation trennen.

In einem weiteren Schritt lassen sich die so erhaltenen Halogenide V leicht zu entsprechenden α-Wasserstoffperfluoralkyl-aromaten der Formel V, in der X Wasserstoff bedeutet, reduzieren. Die Reduktion kann durch Umsetzung mit Wasserstoff über Edelmetallkatalysatoren, wie Platin auf Aktivkohle, oder einfacher durch thermische Reaktion des Halogenids V mit einer organischen, Wasserstoff abgebenden Verbindung, wie reaktiven Alkylaromaten, wie Tetrahydronaphthalin oder Diphenylmethan, erfolgen. Dazu wird die zu reduzierende Verbindung mit dem Alkylaromaten auf Temperaturen von in der Regel 160°C bis 220°C erhitzt. Diese Reduktion kann auch in einem Schritt, ausgehend von den Phosphoniumsalzen I, durchgeführt werden, da unter diesen Reaktionsbedingungen rasch eine Umwandlung in das Halogenid V erfolgt. Das Reaktionsprodukt läßt sich z.B. durch Destillation isolieren. Mit dieser Reaktionsfolge wird ein bequemer Zugang zu aromatischen Verbindungen der Struktur V mit fluorierten Seitenketten zur Verfügung gestellt. Diese Verbindungen sind interessante Zwischenprodukte, die bisher nur in aufwendigen und mehrstufigen Synthesen herstellbar waren.

Die Strukturen der Verbindungen gemäß den Beispielen 1 bis 7 und deren wichtigste physikalische Daten sind in der Tabelle zusammengefaßt. Soweit in den Beispielen zum Umkristallisieren Lösungsmittelgemische verwendet wurden, wurde ein Volumenverhältnis von 1 : 1 angewandt, wobei jedoch durch Veränderung des Verhältnisses noch Optimierungen, auch hinsichtlich der Ausbeute, möglich sind.

**Beispiele 1-7 – Verbindungen der Formel (I)**

1) In einen Rundkolben wurden unter Ausschluß von Feuchtigkeit bei ca. –70°C zu einer Lösung von 26,5 g (0,25 mol) Benzaldehyd in 150 ml $CH_2Cl_2$ 41 g (0,27 mol) Trifluormethylbromid einkondensiert. Anschließend wurden innerhalb einer halben Stunde 66,7 g (0,27 mol) Phosphorigsäuretrisdiäthylamid unter Rühren zudosiert. Nach 4 Stunden bei –70°C war der Aldehyd laut IR-Spektroskopie umgesetzt. Dann wurde das Reaktionsgemisch langsam auf Raumtemperatur erwärmt und das Lösungsmittel unter vermindertem Druck verdampft. Nach der Umkristallisation des Rohproduktes aus Methyl-t-butyläther/Essigsäureäthylester erhielt man 97,2 g (77% Ausbeute) farblose Kristalle von (1-Phenyl-2, 2, 2-trifluoräthoxy)-tris (diäthylamino)phosphoniumbromid vom Schmelzpunkt 129°C.

2) In einen Rundkolben wurden unter Rühren und Ausschluß von Feuchtigkeit bei ca. 0°C zu einer Lösung von 39,6 g (0,25 mol) 2-Chlor-6-fluorbenzaldehyd in 150 ml Buttersäurenitril 61,7 g (0,25 mol) Phosphorigsäuretrisdiäthylamid gegeben. Dann wurden bei 20 bis 25°C 42,5 g (0,28 mol) Trifluormethylbromid in dem Maße in die Lösung geleitet, wie es verbraucht wurde. Nach ca. 4 Stunden war die Umsetzung vollständig. Das Reaktionsgemisch wurde zweimal mit je 200 ml Hexan extrahiert. Der Extraktionsrückstand wurde unter vermindertem Druck vom restlichen Lösungsmittel befreit. Nach der Umkristallisation des erhaltenen Rückstandes (123 g) aus Tetrahydrofuran wurden 104 g (75% Ausbeute) [1-(2-Chlor-6-fluorphenyl)-2,2,2-trifluoräthoxy]-tris(diäthylamino)phosphoniumbromid in Form von farblosen hygroskopischen

Kristallen vom Schmelzpunkt 123 bis 124°C erhalten.

3) In einen Rundkolben wurden unter Ausschluß von Feuchtigkeit bei ca. −70°C zu einer Lösung von 34,3 g (0,25 mol) ω,ω,ω-Trifluoracetophenon in 150 ml $CH_2Cl_2$ 41 g (0,27 mol) Trifluormethylbromid einkondensiert. Bei dieser Temperatur wurden dann unter gutem Rühren 66,7 g (0,27 mol) Phosphorigsäuretrisdiäthylamid innerhalb von einer Stunde zugetropft. Nach weiteren 6 Stunden wurde das Reaktionsgemisch langsam auf Raumtemperatur erwärmt und zweimal mit je 200 ml Hexan extrahiert. Der Extraktionsrückstand wurde unter vermindertem Druck vom restlichen Lösungsmittel befreit. Nach der Umkristallisation des erhaltenen Rückstands (161 g) aus Tetrahydrofuran/Aceton wurden 121 g (85% Ausbeute) [1-Phenyl-2,2,2-trifluor-1-(trifluormethyl)-äthoxy]-tris(diäthylamino)phosphoniumbromid in Form farbloser Kristalle vom Schmelzpunkt 168°C erhalten.

Die Verbindungen gemäß den in der Tabelle aufgeführten Beispielen 4 bis 7 wurden nach dem Verfahren gemäß Beispiel 3 hergestellt.

**Beispiele 8-11 – Verbindungen der Formel (V)**

8) In einer Destillationsapparatur wurden 50 g (0,1 mol) (1-Phenyl-2,2,2-trifluoräthoxy)-tris(diäthylamino)phosphoniumbromid (erhalten nach Beispiel 1) geschmolzen und kurz auf 140°C erhitzt. Bei der anschließenden Destillation erhielt man 23,5 g (99% Ausbeute) (1-Brom-2,2,2-trifluoräthyl)-benzol (Kp. 68°C/15 mbar) sowie 24,9 g (95% Ausbeute) Phosphorsäuretrisdiäthylamid als Begleitprodukt.

9) In einem Rundkolben mit Rückflußkühler wurden 53,2 g (0,1 mol) [1-(4-Methoxyphenyl)-2,2,2-trifluoräthoxy]-tris(diäthylamino)phosphoniumbromid (Produkt aus Beispiel 5) in 80 ml Methylisobutylketon 10 min unter Rückfluß erhitzt. Bei der anschließenden Destillation erhielt man 22,2 g (83% Ausbeute) 1-(1-Brom-2,2,2-trifluoräthyl)-4-methoxybenzol (Kp. 108 bis 110°C/8 mbar).

10) In einer Destillationsapparatur wurde ein Gemisch aus 50 g (0,1 mol) (1-Phenyl-2,2,2-trifluoräthoxy)-tris(diäthylamino)phosphoniumbromid gemäß Beispiel 1 und 40 g (0,3 mol) Tetrahydronaphthalin 2 Stunden auf ca. 200°C erhitzt. Nach etwa einer Stunde wurde das Reaktionsprodukt langsam über eine kleine Kolonne abdestilliert, wobei die Siedetemperatur am Kolonnenkopf nicht über 140°C stieg. Das restliche Produkt wurde nach beendeter Reaktion bei 40 mbar aus dem Reaktionsgemisch abdestilliert. Bei der erneuten Destillation der vereinigten Fraktionen erhielt man 10,2 g (64% Ausbeute) (2,2,2-Trifluoräthyl)benzol vom Kp. 71 bis 72°C/100 mbar.

11) In einer Destillationsapparatur wurde ein Gemisch aus 57 g (0,1 mol) [1-Phenyl-2,2,2-trifluor-1-(trifluormethyl)-äthoxy]tris(dialkylamino)phosphoniumbromid gemäß Beispiel 3 und 40 g (0,3 mol) Tetrahydronaphthalin 3 Stunden auf ca. 200°C erhitzt. Nach etwa einer Stunde wurde das Reaktionsprodukt langsam über eine Kolonne abdestilliert, wobei die Siedetemperatur am Kolonnenkopf nicht über 160°C stieg. Das restliche Produkt wurde nach beendeter Reaktion bei 20 mbar aus dem Reaktionsgemisch abdestilliert. Nach der erneuten Destillation der vereinigten Fraktionen erhielt man 12,8 g (56% Ausbeute) 1,1,1,3,3,3-Hexafluor-2-phenylpropan (Kp. 83 bis 84°C/ 100 mbar).

**Vergleichsbeispiel**

Die Tatsache, daß bei der Umsetzung der erfindungsgemäß verwendeten Ausgangsprodukte zunächst ein salzartiges Addukt der Formel VI gebildet wird, das sich von den erfindungsgemäßen Verbindungen I durch seine Reaktivität mit Carbonsäurehalogeniden unter Bildung von Estern unterscheidet, wird durch den folgenden Vergleichsversuch zum Beispiel 3 belegt:

Wie im Beispiel 3 wurden die gleichen Mengen ω,ω,ω-Trifluoracetophenon, $CH_2Cl_2$, Trifluormethylbromid und Phosphorigsäuretrisdiäthylamid zusammengegeben. Vier Stunden nach Beendigung der Zugabe des Phosphorigsäuretrisdiäthylamids wurden 35,1 g (0,25 mol) Benzoylchlorid zugegeben. Anschließend wurde noch 2 Stunden bei ca. −70°C gerührt. Nach Erwärmen der Reaktionsmischung auf Raumtemperatur wurden 300 ml Hexan zugegeben. Nach Phasentrennung wurde die untere Phase nochmals sorgfältig mit Hexan extrahiert. Die vereinigten Hexanphasen wurden eingeengt und unter vermindertem Druck destilliert. Es wurden 32,5 g (75%) 1, 1, 1, 3, 3, 3-Hexafluor-2-phenyl-propyl-2-benzoat mit Siedepunkt 96 bis 97°C/0,1 mbar erhalten.

| C | H | F | $^{19}F$-NMR [ppm] |
|---|---|---|---|
| (ber.) | (ber.) | (ber.) | $CF_3$ |
| gef. | gef. | gef. | |
| (55,18) | (2,89) | (32,74) | -70,5 |
| 55,0 | 2,9 | 32,7 | |

In einem anderen Versuch wurden in einem Rundkolben 11,4 g (0,02 mol) Phosphoniumsalz aus Beispiel 3 und 2,8 g (0,02 mol) Benzoylchlorid in 50 ml $CH_2Cl_2$ gerührt. Selbst nach zweistündigem Erhitzen unter Rückfluß lagen die beiden Ausgangsprodukte unverändert nebeneinander vor ; die Bildung des zuvor beschriebenen Esters wurde nicht beobachtet.

**Tabelle**

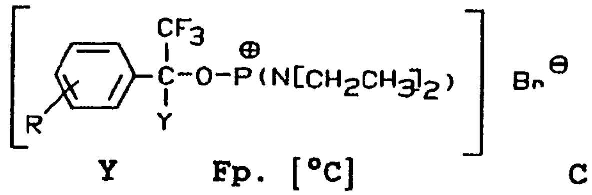

| Bsp. | R | Y | Fp. [°C] (umkrist. aus) | C (ber) gef. | H (ber) gef. | F (ber) gef. | $^{19}$F-NMR [ppm] $-CF_3$ ($CDCl_3$) | Ausbeute |
|---|---|---|---|---|---|---|---|---|
| 1 | H | H | 129 (EE/MtBE) | (47,81) 47,7 | (7,22) 7,4 | (11,34) 10,7 | -76,4 | 77 % |
| 2 | 2-Cl, 6-F | H | 123-4 (THF) | (43,3) 43 | (6,18) 6,1 | (13,7) 13,6 | -75,4 | 75 % |
| 3 | H | $CF_3$ | 168 (THF/Aceton) | (44,22) 43,8 | (5,88) 6,1 | (19,98) 19,9 | -71,2 | 85 % |
| 4 | 4-$CH_3$ | H | 148 (THF) | (48,84) 49,2 | (7,42) 7,2 | (11,03) 11,0 | -76 | 71 % |
| 5 | 4-$OCH_3$ | H | 117-8 (Aceton) | (47,37) 47,1 | (7,19) 7,1 | (10,7) 10,6 | -76,6 | 81 % |
| 6 | 3,4$(CH_3)_2$ | $CF_3$ | 118-9 (MiBK) | (46,16) 46,2 | (6,57) 6,5 | (19,05) 18,7 | -71,0 | 84 % |
| 7 | H | $C_2F_5$ | 137 (MiBK) | (42,59) 43,2 | (5,64) 5,6 | (24,5) 24,4 | -66,1 | 61 % |

EE = Essigsäureäthylester
MtBE = Methyl-t.-butyläther
THF = Tetrahydrofuran
MiBK= Methylisobutylketon

## Ansprüche

1. 1-Arylalkoxy-tris(dialkylamino)phosphonium-Salze der Formel I

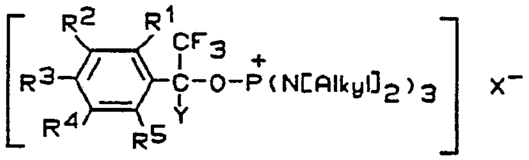

(I)

worin die Reste $R^1$ bis $R^5$ gleich oder verschieden sind und Wasserstoff, Alkyl mit 1 bis 6 Kohlenstoffatomen, das perfluoriert sein kann, Halogen, Alkoxy oder Alkylthio mit jeweils 1 bis 6 Kohlenstoffatomen sind, jedoch nicht mehr als drei der Reste $R^1$ bis $R^5$ eine andere Bedeutung als Wasserstoff haben, Y Wasserstoff oder einen Perfluoralkylrest $C_nF_{2n+1}$ mit 1 bis 6 Kohlenstoffatomen bedeutet, X Brom oder Jod ist und Alkyl für einen Alkylrest mit 1 bis 3 Kohlenstoffatomen steht.

2. Phosphoniumsalze nach Anspruch 1, dadurch gekennzeichnet, daß am aromatischen Ring höchstens zwei der Reste $R^1$ bis $R^5$ eine andere Bedeutung als Wasserstoff haben.

3. Phosphoniumsalze nach Anspruch 1 oder 2, dadurch gekennzeichnet, daß die Alkyl-, Alkoxy- und Alkylthiosubstituenten am aromatischen Ring zusammen höchstens 6, insbesondere höchstens 4 Kohlenstoffatome enthalten.

4. Verfahren zur Herstellung von 1-Arylalkoxy-tris(dialkylamino)phosphoniumsalzen der Formel I, in der $R^1$ bis $R^5$, X und Y die in einem oder mehreren der Ansprüche 1 bis 3 genannten Bedeutungen haben, dadurch gekennzeichnet, daß man aromatische Carbonylverbindungen der allgemeinen Formel II

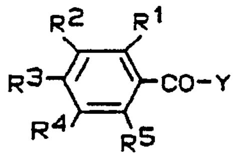

(II)

mit Trifluormethylhalogeniden der allgemeinen Formel $CF_3X$ (III), worin X Brom oder Jod ist, und Phosphorigsäuretrisdialkylamiden der allgemeinen Formel $P(N[Alkyl]_2)_3$ (IV) umsetzt.

5. Verfahren nach Anspruch 4, dadurch gekennzeichnet, daß die Umsetzung bei Temperaturen von etwa −100°C bis +50°C, insbesondere von −80 bis +20°C durchgeführt wird.

6. Verfahren nach Anspruch 4 oder 5, dadurch gekennzeichnet, daß die Umsetzung ohne Anwendung von Überdruck erfolgt.

7. Verfahren nach Anspruch 4 oder 5, dadurch gekennzeichnet, daß die Umsetzung bei höherem als Atmosphärendruck und bei einer Temperatur durchgeführt wird, die oberhalb der Siedetemperatur (bei Atmosphärendruck) des Trifluormethylhalogenids liegt.

8. Verfahren nach einem oder mehreren der Ansprüche 4 bis 7, dadurch gekennzeichnet, daß die Umsetzung unter wasserfreien Bedingungen in Gegenwart eines gegenüber den Reaktionsteilnehmern inerten Lösungs- oder Verdünnungsmittels durchgeführt wird.

9. Verfahren nach einem oder mehreren der Ansprüche 4 bis 8, dadurch gekennzeichnet, daß die anderen Reaktionsteilnehmer in einer zur Carbonylverbindung II mindestens stöchiometrischen Menge und höchstens in einem Überschuß bis zu 25% eingesetzt werden.

10. Verwendung von 1-Arylalkoxy-tris(dialkylamino)phosphoniumsalzen der Formel I, wie in Anspruch 1 geseigt, zur Herstellung von aromatischen Verbindungen mit teilfluorierten Seitenketten der allgemeinen Formel V

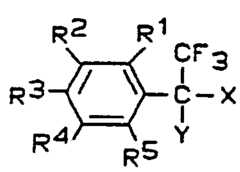

(V),

worin $R^1$ bis $R^5$, X und Y die in einem oder mehreren der Ansprüche 1 bis 3 genannten Bedeutungen haben,

X jedoch auch Wasserstoff sein kann.

## Claims

1. 1-Arylalkoxy-tris(dialkylamino)phosphonium salts of the formula I

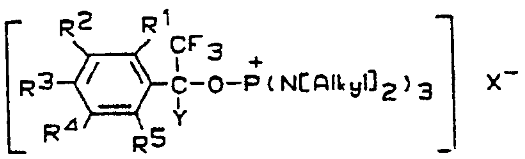

(I)

wherein the substituents $R^1$ to $R^5$ are equal or different and represent hydrogen, alkyl having from 1 to 6 carbon atoms which may be perfluorinated, halogen, alkoxy or alkylthio, each having from 1 to 6 carbon atoms, Y represents hydrogen or a perfluoroalkyl group $C_nF_{2n+1}$ having from 1 to 6 carbon atoms, X represents bromine or iodine and "Alkyl" represents an alkyl group of 1 to 3 carbon atoms, at most 3 of the groups $R^1$ to $R^5$ having however a meaning other than hydrogen.

2. Phosphonium salts according to claim 1, wherein at most 2 of the groups $R^1$ to $R^5$ have a meaning other than hydrogen.

3. Phosphonium salts as claimed in claim 1 or 2, wherein the alkyl, alkoxy and alkylthio substituents attached to the aromatic nucleus altogether contain at most 6, preferably at most 4 carbon atoms.

4. A process for the preparation of 1-aryloxy-tris(dialkylamino)phosphonium salts of the formula I in which $R^1$ to $R^5$, X and Y have the meaning indicated in one or more of claims 1 to 3, characterized in that aromatic carbonyl compounds of the general formula II

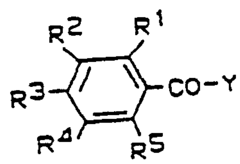

(II)

are reacted with trifluoromethyl halides of the general formula $CF_3X$ (III) and a trisdialkylamide of phosphorous acid of the formula $P(N[Alkyl]_2)_3$ (IV).

5. A process as claimed in claim 4, wherein the reaction is carried out at a temperature in the range of from about −100°C to +50°C, in particular of from −80°C to +20°C.

6. A process as claimed in claim 4 or 5, wherein the reaction is carried out without application of superatmospheric pressure.

7. A process as claimed in claim 4 or 5, wherein the reaction is carried out at a pressure higher than ambient pressure and at a temperature which is above the boiling point (at ambient pressure) of the trifluoromethyl halide.

8. A process as claimed in one or more of claims 4 to 7, wherein the reaction is carried out under anhydrous conditions in the presence of a solvent or diluent inert towards the reactants

9. A process as claimed in one or more of claims 4 to 8, wherein in relation to carbonyl compound II, the other reactants are applied in at least a stoechiometric amount and at most an amount exceeding the stoechiometric amount by 25%.

10. Use of 1-aryloxy-tris(dialkylamino)phosphonium salts of the formula I as shown in claim 1 to yield a compound of the general formula V

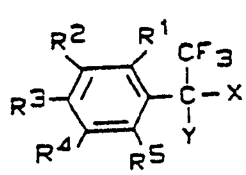

(V),

wherein $R^1$ to $R^5$, X and Y have the meaning indicated in one or more of claims 1 to 3, wherein X however may also be hydrogen.

## Revendications

1. Sels d' 1-(arylalcoxy)-tris-(dialkylamino)-phosphoniums répondant à la formule I :

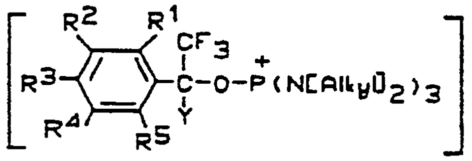

(I)

dans laquelle :
$R^1$ à $R^5$ représentent chacun, indépendamment les uns des autres, l'hydrogène, un alkyle contenant de 1 à 6 atomes de carbone, qui peut être perfluoré, un halogène, un alcoxy ou un alkylthio contenant chacun de 1 à 6 atomes de carbone, mais au plus 3 des symboles $R^1$ à $R^5$ pouvant avoir une signification autre que l'hydrogène,
Y représente l'hydrogène ou un radical perfluoralkyle $C_nF_{2n+1}$ contenant de 1 à 6 atomes de carbone,
X représente le brome ou l'iode et
"Alkyl" représente un radical alkyle contenant de 1 à 3 atomes de carbone.

2. Sels de phosphonium selon la revendication 1, caractérisés en ce que, sur le noyau aromatique, au plus deux des symboles $R^1$ à $R^5$ ont une signification autre que l'hydrogène.

3. Sels de phosphonium selon l'une des revendications 1 et 2, caractérisés en ce que les substituants alkyles, alcoxy et alkylthio sur le noyau aromatique contiennent ensemble au plus 6 atomes de carbone, plus particulièrement au plus 4.

4. Procédé pour préparer des sels d' 1-(arylalcoxy)-tris(dialkylamino)-phosphonium de formule I dans laquelle $R^1$ à $R^5$, X et Y ont les significations qui leur ont été données dans l'une quelconque des revendications 1 à 3, procédé caractérisé en ce qu'on fait réagir des composés carbonyliques aromatiques de formule générale II :

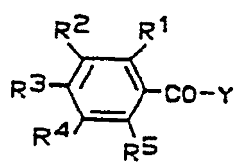

(II)

avec des halogénures de trifluorométhyle répondant à la formule :

$CF_3X$ (III)

dans laquelle X représente le brome ou l'iode, et des tris-(dialkylamides) de l'acide phosphoreux de formule générale :

$P(N[Alkyl]_2)_3$ (IV).

5. Procédé selon la revendication 4, caractérisé en ce que la réaction est effectuée à des températures d'environ –100 à +50°C, plus particulièrement de –80 à +20°C.

6. Procédé selon l'une des revendications 4 et 5, caractérisé en ce que la réaction est effectuée sans application d'une surpression.

7. Procédé selon l'une des revendications 4 et 5, caractérisé en ce que la réaction est effectuée sous une pression supérieure à la pression atmosphérique et à une température supérieure à la température d'ébullition (sous la pression atmosphérique) de l'halogénure de trifluorométhyle.

8. Procédé selon l'une quelconque des revendications 4 à 7, caractérisé en ce que la réaction est effectuée dans des conditions anhydres, en présence d'un solvant ou diluant inerte à l'égard des corps participant à la réaction.

9. Procédé selon l'une quelconque des revendications 4 à 8, caractérisé en ce que les autres corps participant à la réaction sont mis en jeu en une quantité au moins stoechiométrique par rapport au composé carbonylique (II) et au plus en un excès allant jusqu'à 25%.

10. Application de sels d' 1-(arylalcoxy)-tris-(dialkylamino)-phosphonium de formule I comme montré dans la revendication 1, à la préparation de composés aromatiques porteurs de chaînes latérales partiellement fluorées qui répondent à la formule générale V :

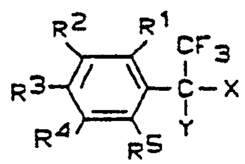

(V)

dans laquelle $R^1$ à $R^5$, X et Y ont les significations qui leur ont été données dans l'une quelconque des revendications 1 à 3, le symbole X pouvant toutefois représenter aussi l'hydrogène.